# EUROPEAN PATENT APPLICATION

(11) **EP 3 167 795 A1**
(43) Date of publication of application: **17.05.2017**
(21) Application number: 15194115.0
(22) Date of filing: 11.11.2015
(51) Int. Cl.: A61B 5/00, A61B 5/11, A61B 3/113

(54) **ARRANGEMENT AND METHOD FOR MONITORING AN EPILEPSY PATIENT**

(71) Applicant: Coche, Tamara, 4052 Basel (CH)
(72) Inventor: Coche, Tamara, 4052 Basel (CH)
(74) Representative: Latscha Schöllhorn Partner AG

(57) **Abstract**

An arrangement (100) for monitoring an epilepsy patient (301) during night-time, comprises: a resting location (300) equipped with a camera (302a, 302b) and a microphone (302a, 302b) for continuously gathering visual and acoustic data of the epilepsy patient (301) during night-time, wherein the resting location (300) is in a first time zone; a supervising location equipped with a screen, a speaker and an alarm trigger (200), wherein the supervising location is in a second time zone different from the first time zone; a caregiving location equipped with an alarm device (400), wherein the caregiving location is in the first time zone and in vicinity of the resting location; and a central unit (101) connected to the camera (302a, 302b) and microphone (302a, 302b) of the resting location (300), to the screen and the alarm trigger of the supervising location and to the alarm of the caregiving location. The central unit (101) is arranged to route the visual and acoustic data gathered at the resting location (300) to the screen and speaker at the supervising location. The central unit (101) is arranged to route alarm data from the alarm trigger at the supervising location to the alarm device at the caregiving location. The arrangement (100) allows for providing an improved reliability of detecting a comparably broad range of different types of seizures.

## Description

### Technical Field

The present invention relates to an arrangement for monitoring an epilepsy patient during night-time and more particularly to a respective method.

### Background Art

Approximately one percent of the general population is diagnosed with epilepsy and in about a quarter of the epilepsy patients the condition is not sufficiently managed by medication or by other treatment options so that uncontrolled seizures remain a concern. In particular nightly seizure events are a great risk, as these events may be unnoticed by caregivers and emergency medication cannot be applied. In some cases, seizures are prolonged and not self-terminating. In particular for these patients, undetected seizures may result in serve mental and motor regressions or even in death.

For addressing this problem a broad range of automated epilepsy monitoring system exists today. Commercially available systems and approaches under development can roughly be grouped into four broad categories: (1) Systems assessing electroencephalogram (EEG), electrocardiogram (ECG), movement and skin conductivity data collected from sensors attached the patient's body. (2) Systems assessing data on patient's movements measured with sensors attached the patient's mattress. (3) Systems assessing video and audio signals of the patient. (4) Systems assessing neurological signals collected from invasive sensors.

All of the above systems rely on the automated, algorithm-based detection of seizure events. Even though these systems provide for recognition of many seizure events none of them, however, provides high confidence in the detection of subtle, focal seizures or generalized seizures with limited motor involvement.

Therefore, there is a need for an arrangement or method having an improved reliability for detecting a comparably broad range or essentially all types of seizures.

### Disclosure of the Invention

According to the invention this need is settled by an arrangement as it is defined by the features of independent claim 1, and by a method as it is defined by the features of independent claim 8. Preferred embodiments are subject of the dependent claims.

In particular, the invention deals with an arrangement for monitoring an epilepsy patient during night-time. The arrangement comprises a resting location equipped with a camera and a microphone for continuously gathering visual and acoustic data of the epilepsy patient when sleeping, wherein the resting location is in a first time zone. It further comprises a supervising location equipped with a screen, a speaker and an alarm trigger, wherein the supervising location is in a second time zone different from the first time zone. The arrangement also includes a caregiving location equipped with an alarm device, wherein the caregiving location is in the first time zone and in vicinity of the resting location. Finally, the arrangement includes a central unit connected to the camera and microphone of the resting location, to the screen and the alarm trigger of the supervising location and to the alarm of the caregiving location. The central unit is arranged to route the visual and acoustic data gathered at the resting location to the screen and speaker at the supervising location. It further is arranged to route alarm data from the alarm trigger at the supervising location to the alarm device at the caregiving location.

The term "vicinity" in connection with the caregiving and resting locations can relate to being close enough for timely reaching or accessing the epileptic patient once an alarm is executed. For example, the caregiving and resting locations can be in different rooms of the same building.

The connections between the central unit and the camera, the microphone, the alarm trigger and the alarm device can be established via the Internet. Thereby, the camera can be an Internet protocol camera (IP camera) allowing transfer of its data via the Internet. The camera can include the microphone and provide the visual and acoustic data in one single data stream. It can further be a night vision camera.

The term "caregiver" as used herein can relate to a person attending the patient or epilepsy patient, performing emergency measures on the patient and communicating with the system using the given infrastructure such as a user interface. The caregiver can particularly be a person at the caregiving location.

The term "monitor" as used herein can relate to a human expert or medically trained person monitoring patient data at a remote location and particularly at the supervising location. The monitor can assess if there are seizure events, can trigger alerts via the alert trigger and can log event reports, e.g. using a user interface. A monitor may be assigned to a patient as primary monitor or have the role of a secondary monitor. In its role as primary monitor, the monitor may track live patient data and may trigger alerts to the caregiver. In its role as secondary monitor, the monitor may track either live patient data as a back-up or follow previously recorded patient data for training and quality assurance purposes. One monitor can perform simultaneously the roles of a primary and secondary monitor.

The term "seizure event" as used herein can relate to an episode of an epileptic fit of the patient that needs medical attention.

The term "patient data stream" as used herein can relate to a collection of data continuously streamed from the resting location. This data comprises visual and acoustic or video and audio data. Optionally, this data may additionally comprise parameters such as patient's body temperature, skin conductivity, EEG and ECG readings and measures of body movements.

The term "event" as used herein can relate to the observation on the patient data, which meets reporting or alert criterions.

In this connection, the term "alert criterion" can relate to a set of patient-specific conditions determining the requirement of sending an alert to the caregiver. The conditions may be defined on the basis of body parameters such as jerky movements of the patient's limbs or the status of the system such as a connection down.

Also in this connection, the term "reporting criterion" can relate to a set of patient-specific conditions determining the requirement of logging an event report in the system. The conditions may be defined on the basis of body parameters such as the patient being awake for a prolonged period during the night or the status of the system such as the connection being down.

The term "alert" as used herein can relate to a notification sent from the system to the caregiver that a seizure event may have occurred. Alerts may be delivered to the caregiver outside the system, if some components of the system are down such as in case the connection to the patient's home is down, an alert may be delivered by telephone or the caregiver cannot be contacted by the system.

The term "secondary alert" as used herein can relate to a notification sent from the secondary monitor to the system. The secondary alert may be forwarded to the primary monitor and/or may be recorded in the system for quality assurance and training purposes.

The term "event report" as used herein can relate to the recording of an event in the system by the monitor. An event report may comprise recorded patient data with time stamp information and a written report by the monitor.

The term "alert receipt" as used herein can relate to a notification sent by the caregiver to the system that an alert has been received. Alternatively, it can be a notification sent by the primary monitor to the secondary monitor that an alert has been received.

The term "alert feedback" can relate to a notification sent by the caregiver on the accuracy of the previously triggered alert. Alternatively, it can be a notification sent from the central unit to the secondary monitor on the accuracy of an alert triggered on recorded patient data.

The term "missing alert notification" as used herein can relate to a notification sent by the caregiver to report a seizure event that has remained undetected by the system. Alternatively, it can be a notification sent by the central unit that a seizure event in recorded data remained undetected by the secondary monitor.

In use of the arrangement according to the invention the epilepsy patient sleeps at the resting location. Thereby, he is observed by the camera and the microphone which create a continuous stream of visual and acoustic data. This patient data stream is transferred via the control unit to the supervising location. There, the visual data can be displayed on the screen and the acoustic data can be played by the speaker. At the supervising location a medically trained person, i.e. the monitor, watches the visual data on the screen and listens the acoustic data provided by the speaker. The monitor can particularly be trained in understanding the different appearances of epileptic attacks, i.e. seizure events. Thereby, the monitor of medically trained person can particularly be sensitized to detect subtle, focal seizures or generalized seizures with limited motor involvement. For example, the monitor can be trained to track the eyes of the epilepsy patient. When the eyes do behave in particular manner, such as upward eye deviations and other unusual ocular movements, the monitor can identify a situation which might be a particular epileptic attack, i.e. a seizure event.

As soon as the monitor detects or identifies a seizure event he provides an alarm signal by operating the alarm trigger. The central unit routes the respective alarm signal to the caregiving location where the alarm device provides an alarm. Thereby, the caregiver gets informed about the seizure event and can take appropriate measures to the epilepsy patient.

Since the monitor is a medically trained person a comparably high level of patient observation can be achieved. In particular, compared to known automated systems this allows for essentially increasing the quality of supervision of the epilepsy patient and to identifying seizure events which cannot satisfyingly be identified by such automated system. Besides of the positive effect such improved supervision has for the patient itself it also improves the situation for the caregiver. For example, the caregiver may be the parents of the epilepsy patient. In such a situation the improved quality of supervision allows the caregiver or parents to rest and, e.g., also to sleep, wherein it can be trusted that they will be alarmed as soon as a seizure event happens.

Furthermore, since the supervision location is in another time zone than the resting location the monitors can supervise the patient during daytime wherein the patient and caregiver are at night time. Like this the quality of observation can further be improved since the monitor usually is less tired during day-time.

Thus, the arrangement according to the invention allows for providing an improved reliability of detecting a comparably broad range of different types of seizures.

Preferably, the supervising location is equipped with a sensor unit for gathering sensor data, the supervising location is equipped with a sensor data presentation unit, the central unit is connected to the sensor unit of the resting location and to the sensor data presentation unit of the supervising location and the central unit is arranged to route the sensor data gathered at the resting location to the sensor data presentation unit at the supervising location. The sensor presentation unit can be the screen of the supervising location. By augmenting the data gathered from the patient while he is sleeping by other data sensed by the sensing unit the patient data stream can provide for additional parameters of the patient behaviour and condition. Like this, the quality of observation can be further improved.

Thereby, the sensor unit preferably comprises any of a body temperature sensor, a skin conductivity sensor, an electroencephalography sensor, an electrocardiography sensor or a combination thereof. Such sensors allow for gathering particular additional data which might be relevant for detecting a seizure event.

Preferably, the central unit is arranged to monitor a status of the camera, microphone, screen speaker, alarm trigger, alarm device and the connection in between, to identify a faulty status and to trigger an alarm on the alarm device at the caregiving location when the faulty status is identified. Like this, the status of the arrangement or system can also be supervised such that any inconsistencies in the system itself can be identified and respective measures can be taken. This allows decreasing the risk of a malfunctioning of the arrangement.

Preferably, the arrangement further comprises a secondary supervising location equipped with a screen, a speaker and an alarm trigger, wherein the secondary supervising location is in a third time zone different from the first time zone, wherein the central unit is connected to the screen and the alarm trigger of the secondary supervising location, the central unit is arranged to route the visual and acoustic data gathered at the resting location to the screen and speaker at the secondary supervising location, and the central unit is arranged to route alarm data from the alarm trigger at the secondary supervising location to the alarm device at the caregiving location.

The second and third time zone may be the same. Such double or multiple patient observations allows for even further increasing the quality and accuracy of the epilepsy patient observation.

Preferably, the central unit comprises a database and is arranged to store the visual and acoustic data gathered at the resting location and the alarm data routed from the alarm trigger at the supervising location to the alarm device at the caregiving location. Such a database allows for gathering a history of the observation of the patient and for reporting the observation.

Preferably, the arrangement comprises at least one further resting location equipped with a camera and a microphone for continuously gathering visual and acoustic data of at least one further epilepsy patient when sleeping, at least one further caregiving location equipped with an alarm device, wherein the at least one further caregiving location is vicinity of the at least one further resting location, and the central unit is arranged to route the visual and acoustic data gathered at the at least one further resting location to the screen and speaker at the supervising location and to route alarm data from the alarm trigger at the supervising location to the alarm device at the at least one further caregiving location. Like this the system can be set up to provide observation services for plural patients and caregivers at the same time.

A further aspect of the invention is a method of monitoring an epilepsy patient during night-time by means of an arrangement as described above. The method comprises the steps of: the epilepsy patient sleeping at the resting location; the central unit routing the visual and acoustic data of the epilepsy patient gathered at the resting location to the screen and speaker at the supervising location; a medically trained person observing the screen and the speaker at the supervising location; the medically trained person activating the alarm trigger when identifying a seizure event by interpreting the visual and acoustic data provided by the screen and speaker at the supervising location; a caregiving person receiving an alarm provided by the alarm device at the caregiving location upon the alarm trigger activated at the supervising location; and the caregiving person transfers to the resting location and attends the epilepsy patient.

The method according to the invention allows for efficiently implementing the effects and benefits described above in connection with arrangement according to the invention.

Preferably, the method further comprises the step of predefining an alert criterion, wherein the medically trained person activates the alarm trigger when the visual and acoustic data provided by the screen and speaker at the supervising location fulfil the alert criterion. Further, the method preferably further comprises the step of the medically trained person activates the alarm trigger when identifying a faulty status of the arrangement.

The method according to the invention or at least portions thereof may be implemented by one or multiple computer program. Such computer programs can be executed on a server computer as well as on computer devices operated by the caregivers and monitors.

### Brief Description of the Drawings

The arrangement and method according to the invention are described in more detail hereinbelow by way of an exemplary embodiment and with reference to the attached drawings, in which:
- Fig. 1: shows a schematic overview of an epilepsy monitoring system as an embodiment of the arrangement according to the invention;
- Fig. 2: shows components of the epilepsy monitoring system of Fig. 1 which are arranged in a patient's home;
- Fig. 3: shows a user interface for a caregiver of the epilepsy monitoring system of Fig. 1;
- Fig. 4: shows a user interface for a monitor of the epilepsy monitoring system of Fig. 1;
- Fig. 5: shows a flow chart of a monitoring initiation in an embodiment of a method according to the invention using the epilepsy monitoring system of Fig. 1;
- Fig. 6: shows a flow chart of a monitoring of a primary data stream in the method of Fig. 5;
- Fig. 7: shows a flow chart of a monitoring of a secondary data stream in the method of Fig. 5;
- Fig. 8: shows a flow chart of the monitor triggering an alert within the method of Fig. 5; and
- Fig. 9: shows a flow chart of the caregiver receiving the alert within the method of Fig. 5.

### Description of Embodiments

In the following, to avoid repetition in the figures and the descriptions of the various aspects and illustrative embodiments, it should be understood that many features are common to many aspects and embodiments. Omission of an aspect from a description or figure does not imply that the aspect is missing from embodiments that incorporate that aspect. Instead, the aspect may have been omitted for clarity and to avoid prolix description. In this context, the following applies to the rest of this description: If, in order to clarify the drawings, a figure contains reference signs which are not explained in the directly associated part of the description, then it is referred to previous or following description sections. Further, for reason of lucidity, if in a drawing not all features of a part are provided with reference signs it is referred to other drawings showing the same part. Like numbers in two or more figures represent the same or similar elements.

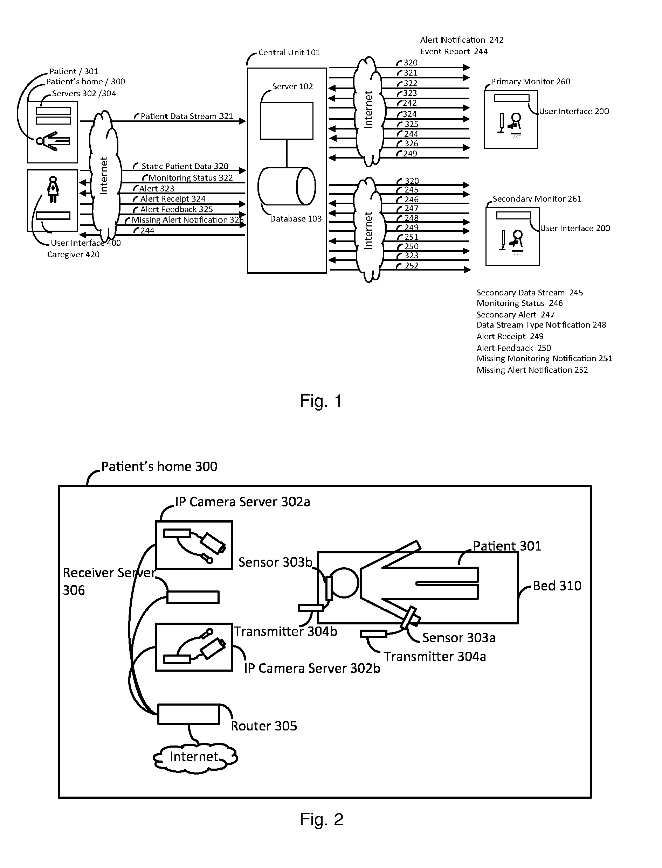

Fig. 1 shows an epilepsy monitoring system 100 as an embodiment of an arrangement according to the invention. The epilepsy monitoring system 100 routes a continuous stream of patient data from the patient's home 300 as a resting location via the Internet and via a central unit 101 of the epilepsy monitoring system 100 to sites of a primary monitor 260 and a secondary monitor 261 as supervising location. The primary and secondary monitor 260, 261 are medically trained personnel such as nurses or the like who make judgments on seizure events happening at the patient's home and who trigger alerts which are routed via the Internet and the central unit 101 to a caregiver 420 at a caregiving location. The patient's 300 home and the caregiving location are in a first time zone such as the Central European Time (CET) zone and the supervising location is in a second time zone such as the Philippine Time (PHT) zone.

The epilepsy monitoring system comprises the central unit 101 for data routing and data storage, a number of components including a camera and a microphone in the patient's home 300, an alarm device implemented in a caregiver user interface 400 executed at the caregiving location for the patient's caregivers 420 as well as a screen, a speaker and an alarm trigger implemented in a monitor user interface 200 at the supervising location for the monitors 260, 261. The epilepsy monitoring system 100 is configured for multiple patients 301 and primary and secondary monitors 260, 261 wherein in Fig. 1 one of each is exemplified.

The continuous stream of patient data 321 from the patient's home 300 is gathered by the camera, the microphone and possibly other sensors about the patient 301 at the patient's home 300 and, thus, at least comprises visual and acoustic data about the patient 301. The patient data stream 321 is transferred to the central unit 101 via the Internet. The central unit 101 has a server 102 for routing various data streams including the patient data stream 321 and a database 103. It stores data from the patient data stream 321 in the database 103 of the central unit 101.

The central unit 101 routes the patient data stream 321 to the primary monitor 260. It further routes a secondary data stream 245 to the secondary monitor 261. The secondary data stream 245 is a combination of live and previously recorded patient data. The routing of the secondary data stream 245 serves two purposes: First - when the secondary data stream 245 constitutes of live data - it facilitates the identification of negligence in the monitoring of the primary data stream or patient data stream 321 when the secondary monitor 261 detects a seizure event which remains unnoticed by the primary monitor 260 or the primary monitor does not respond in a timely fashion to an alert triggered by the secondary monitor 261. Second - when the secondary data constitutes of previously recorded data with known seizure events - the ability of the secondary monitor 261 to identify seizure events can be tested. The central unit 101 randomly switches in the routing of the secondary data stream 245 between live data and previously recorded data of seizure events. The primary data stream or patient data stream 321 is always live.

As mentioned, the communication between the central unit 101, components in the patient's home 300, the caregiver user interface 400 and the monitor user interface 200 is facilitated via the Internet. The epilepsy monitoring system 100 assures the monitoring quality and decision-making quality by making use of alert feedbacks 250 and missing alert notifications 251 as well as simultaneously routing the secondary data stream 245.

The primary information flows in the epilepsy monitoring system 100 shown in Fig. 1 are as follows: An ongoing stream of the patient data 321 is routed via the central unit 101 to the primary monitor 260 who has been assigned as primary for the particular patient 301. The patient data stream 321 is also stored in the database 103.

The primary monitor 260 triggers an alert 323 via the alert trigger in case a seizure event is suspected by inspecting the patient data stream 321 on the monitor user interface 200. The alert 323 is routed via the central unit 101 to the caregiver 420. The caregiver 420 acknowledges the alert 323 by sending an alert receipt 324 that is routed to the primary monitor 260 via the central unit 101. Subsequently, the caregiver sends an alert feedback 325 that is routed by the central unit 101 to the primary monitor 260. In case of an undetected seizure, the caregiver sends a missing alert report 326 that is routed by the central unit 101 to the primary monitor 260.

The central unit 101 also routes the secondary data stream 245 to the secondary monitor 261. The secondary data stream 245 may either be live patient data 321 or a previously recorded data stream of the same or another patient. The secondary monitor 261 triggers an alert 247 if seizure activity is suspected by inspecting the secondary data stream 245. If the secondary data stream 245 is live patient data 321, the epilepsy monitoring system 100 generates an alert notification 242 that is routed by the central unit 101 to the primary monitor 260. If the secondary data stream 245 is previously recorded data, an alert receipt 249 and an alert feedback 250 are sent to the secondary monitor 261 from the central unit 101 automatically.

All alerts 323, 247, alert receipts 324, 249, alert feedbacks 325, 250, missing alert reports 326, 251 are stored in the database 103 of the central unit 101 and are used for assessing the monitoring quality of the overall epilepsy monitoring system 100 and the individual monitors 260, 261.

The components in the patient's home 300 are shown in more detail in in Fig. 2. The minimum configuration comprises one IP camera 302a with pan and tilt functionality positioned close to a patient's bed 310. The IP camera 302a generates the data stream 321 during the monitoring hours specified by the caregiver 420, i.e. typically over night. The IP camera 302a has an optical sensor with night vision and a microphone. The IP camera 302a is connected via a router 305 to the Internet. The pan and tilt functionality can be used by the caregiver 420 or the primary monitor 260 to focus on the patient's 301 body or body parts such as the face. The IP camera 302a may also have zoom functionality.

Optionally the components can additionally comprise one or more additional IP cameras 302b that focus on the patient from a different angle than the IP camera 302a. Further, the components can comprise additional body sensors 303a, 303b which continuously collect body parameters such as patient's heart rate, body temperature, skin conductivity patent reference as well as EEG and ECG readings. Sensor 303a may also measure body movements used to detect seizure events. Data from sensors 303a, 303b are transmitted wirelessly within the patient's home 300 from transmitters 304a and 304b to receiver server 306. The receiver server is connected via the router to the Internet. In embodiments involving additional body sensors 303a, 303b, the patient data stream 321 can be augmented by data collected by these sensors 303a, 303b.

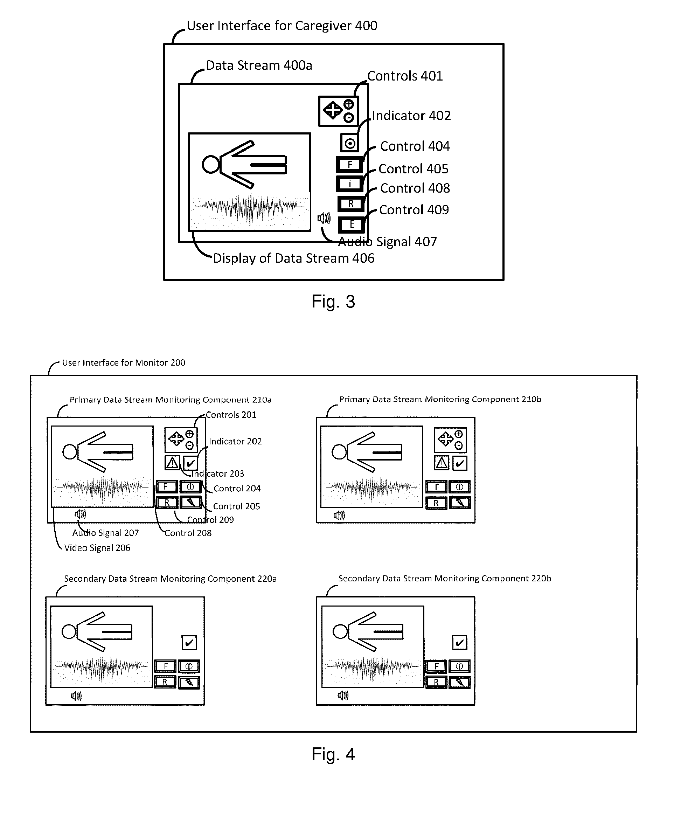

Fig. 3 shows a design of the caregiver user interface 400 running at the caregiving location for the caregivers 420. The caregiver user interface 400 can be implemented as a desktop application on a computer, an application on a mobile device or as a website to be operated within an Internet browser. It comprises a display 406 of the patient data stream 321 in visualized form and parts of the patient data stream 321 are made available as an audio signal 407.

An indicator 402 displays a monitoring status 322. The monitoring status 322 is a feedback from the primary monitor 260 that is routed via the central unit 101. The monitoring status 322 can take values online and offline. The caregiver user interface 400 comprises controls 401 that can be used to configure the patient data stream 321. In particular, the controls 401 can be used to operate the IP cameras' 302a, 302b pan, tilt and zoom functionality. The controls 401 can also be used to configure the volume of the audio signal. Usage of the controls 401 can overwrite configurations made by the primary monitor 260 using the monitor user interface 200.

The caregiver 420 uses control 408 to send an alert receipt 324 to the monitor 260 after an alert has been received. Control 404 is used to provide alert feedback 325 to the monitors 260, 261. The alert feedback 325 can take the values (a) representing seizure correctly identified and (b) representing false positive alert i.e. Monitor falsely identified a seizure. Control 404 is also used to submit a missing alert notification 326 in case the monitors 260, 261 missed to identify a seizure.

Static patient data 320 is provided by the caregivers 420 to the central unit 101 using control 405. Static patient data 320 comprises information on a patient's epilepsy type e.g. generalized or focal epilepsy, patient-specific signs of seizure activity, e.g. ocular movements, lip smacking and motion jerks, criterions for alert triggering, e.g. lip smacking for longer than five minutes, criterions for reports requirements, e.g. patient is awake, specification of the alert procedure primary and alternate contact details, and days and hours during which monitoring is requested.

The caregiver 420 uses control 409 to retrieve event reports 244 logged by the monitors 260, 261. Each monitor 260, 261 is simultaneously assigned to one or multiple primary data streams 321 and one or multiple secondary data streams 245. The monitors use monitor user interface 200 for the monitoring.

The design and functionality of the monitor user interface 200 is shown in Fig. 4. The monitor user interface 200 can be implemented as a desktop application on a computer, an application on a mobile device or as a website to be operated within an Internet browser. The monitor user interface 200 provides one or multiple primary data stream monitoring components 210a, 210b for the monitoring of primary data streams 321 and one or multiple secondary data stream monitoring components 220a, 220b for the monitoring of secondary data streams 245.

The components for the monitoring of the primary data stream 210a, 210b comprises a display of the data stream 321 in visualized form 206 and parts of the data stream are made available as an audio signal 207. The monitor user interface 200 comprises controls 201 that can be used to configure the data stream. In particular, the controls 201 can be used to operate remotely the IP cameras' 302a, 302b pan, tilt and zoom functionality. The controls 201 can also be used to configure the volume of the audio signal. Usage of the controls 201 can overwrite configurations made by the caregiver 420.

The monitor 260, 261 uses control 204 to retrieve static patient data 320 required for the monitoring, triggering alerts 323 and logging event reports 244. The monitor 260, 261 uses control 205 as alert trigger to trigger an alert 323 that is routed by the central unit 101 to the caregiver user interface 400.

Indicator 203 is used to flag an alert notification 242 submitted by the secondary monitor 261. After receiving an alert notification, the primary monitor 260 sends an alert receipt 249 to the secondary monitor 261 using control 209. Indicator 204 is used to flag that the caregiver 420 has sent an alert receipt 324. The monitor 260, 261 uses control 208 to retrieve alert feedbacks 325 and missing alert reports 326 submitted by the caregiver 420.

The components for the monitoring of the secondary data stream 220a, 220b comprises a display of the data stream 245 in visualized form and parts of the data stream are made available as an audio signal. The functionality for the monitoring of the secondary data stream 245 also comprises controls 204, 205 and 208 as well as indicator 202. For the monitoring of the secondary data stream 245 an alert 247 using control 205 is routed via the central unit 101 to the primary monitor 260 where it is displayed as alert notification 242 using indicator 203. For the monitoring of the secondary data stream Indicator 202 notifies the secondary monitor 261 that the primary monitor 260 has sent an alert receipt 249.

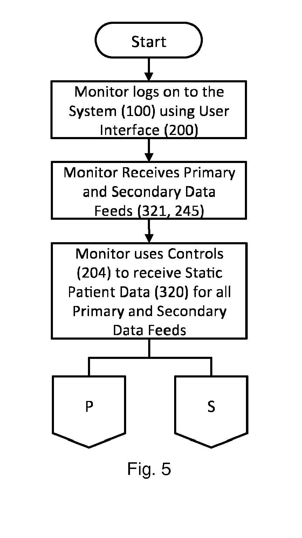

The activities of the monitor 260, 261 for initiating the ongoing monitoring are depicted in Fig. 5. After logging on to the epilepsy monitoring system 100, the monitor 260, 261 receives one or multiple live patient data streams 321 which are routed via the central unit 101 to one of the primary data stream monitoring components 210a, 210b and one or multiple secondary data streams 245 which are routed to the one of the secondary data stream monitoring components 220a, 220b.

For all the primary data streams a monitoring status 322 is set to online. The monitor 260, 261 uses controls 204 to receive static patient data 320 for all primary and secondary data feeds. The monitor 260, 261 continues with the ongoing monitoring of the primary and secondary data streams.

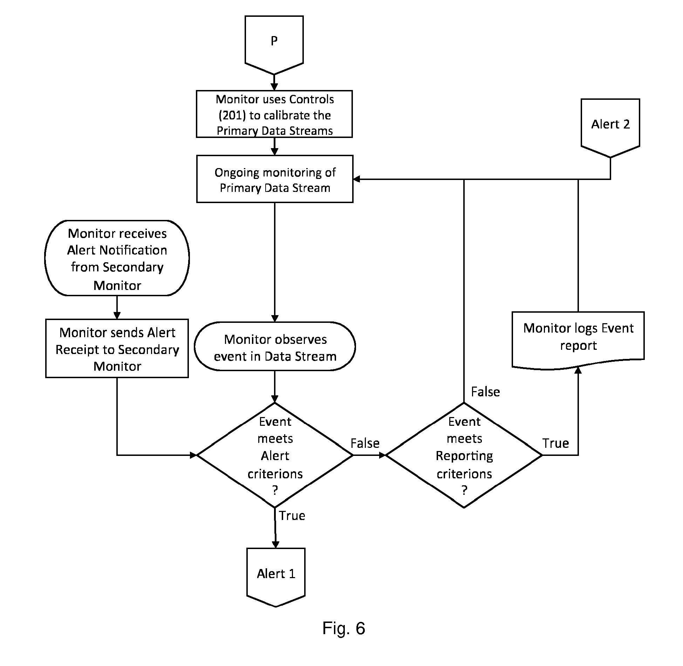

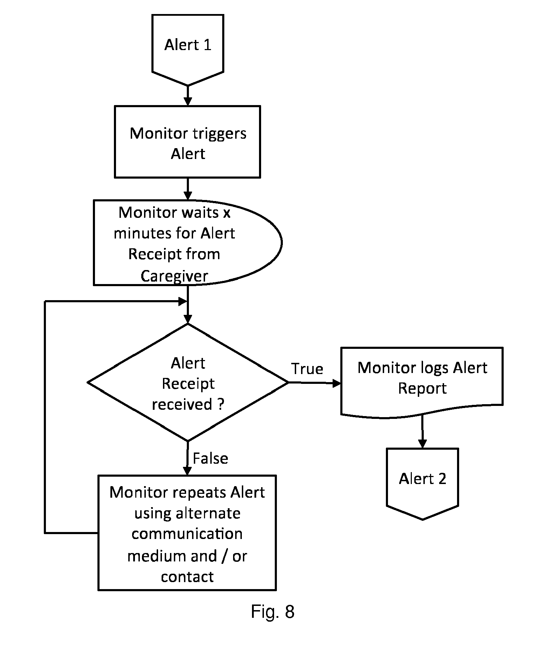

In Fig. 6 the activities in the ongoing monitoring of the primary data stream are shown. The monitor 260, 261 uses controls 201 to calibrate the primary data streams. In case an event is observed by the monitor 260, 261, the static patient data will be used to determine if the event meets the alert criterions. If the event meets the alert criterions, the monitor 260, 261 will proceed with the alert procedure depicted in Fig 8. If the event does not meet the alert criterions, the monitor 260, 261 will assess if the event meets the reporting criterions. If the event meets the reporting criterions, the monitor will log an event report. The primary monitor 260 may also receive an alert notification from the secondary monitor 261. The monitor 260 will send an alert receipt 249 and subsequently assess if the event meets the alert or reporting criterions.

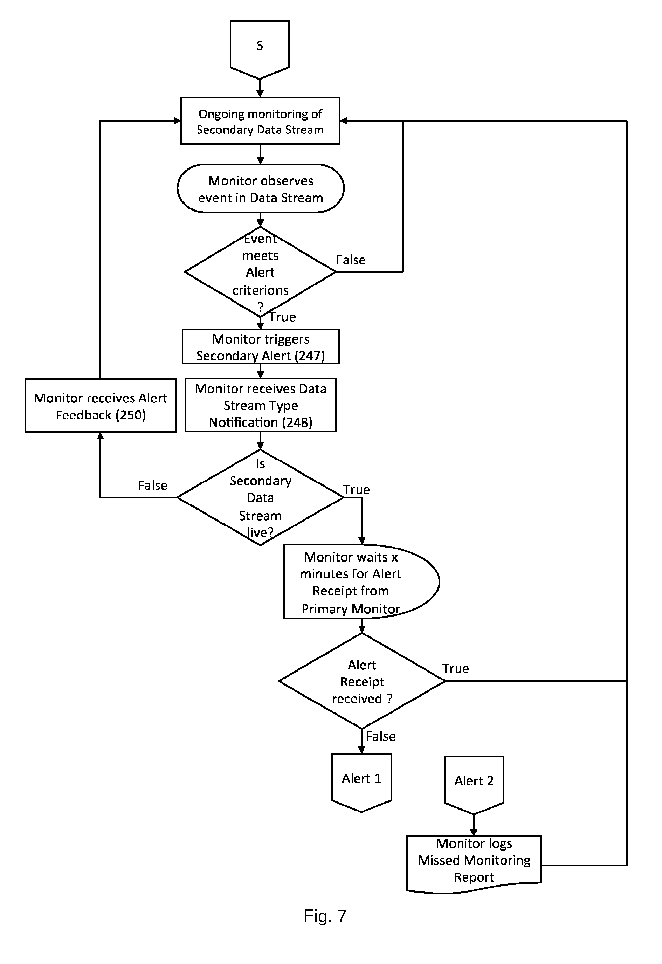

Fig. 7 shows the activities in the ongoing monitoring of the secondary data stream 245. In case an event is observed by the monitor 260, 261, the static patient data will be used to determine if the event meets the alert criterions. If the event meets the alert criterions, the monitor 260, 261 will proceed by triggering a secondary alert 247 that is routed to the central unit 101. Subsequently the monitor 260, 261 receives a data stream type notification 248 providing information if the secondary data stream 245 is live or recorded data. If the secondary data stream 245 is recorded data, the monitor 260, 261 will receive an alert feedback 250 from the epilepsy monitoring system 100. If the secondary data stream 245 is live, the monitor 260, 261 will wait for a specified number of minutes default setting such as x = 2 minutes, for an alert receipt 249 from the primary monitor 260. If the alert receipt 249 has been received, the monitor 260, 261 will continue with the ongoing monitoring activity. If the alert has not been received, the monitor 260, 261 will trigger an alert to the caregiver 323 following the procedure depicted in Fig. 8. Subsequently the monitor 260, 261 logs a missing monitoring report 251.

The procedure for a primary alert 323 triggered by a primary or secondary monitor 260, 261 is shown in Fig. 8. The monitor 260, 261 triggers the alert 323 using control 205 of the monitor user interface 200. The alert 323 is routed via the central unit 101 to the caregiver 420. The monitor 260, 261 now waits for a number of pre-specified minutes default setting such as x = 2 minutes for an alert receipt 324 from the caregiver 420. If an alert receipt has not been received, the monitor 260, 261 will trigger another alert using alternative contact details specified in the static patient data 320. The monitor 260, 261 continues this procedure until an alert receipt has been received. As soon as an alert receipt has been received, the ongoing monitoring activities are resumed.

The caregiver 420 will use control 405 on the caregiver user interface 400 to provide static patient data. The caregiver 420 will use indicator 402 for the monitoring status 322 to check if the patient 301 is monitored by the epilepsy monitoring system 100.

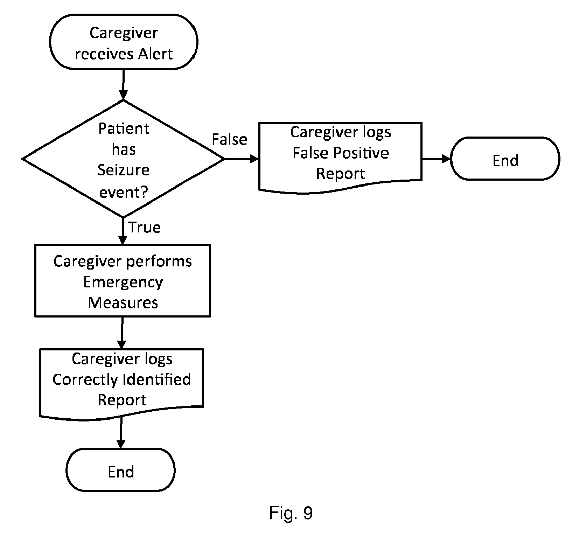

The activities of the caregiver 420 in the case of an alert are shown in Fig. 9. Following the receipt of an alert the caregiver 420 will assess if the patient 301 has a seizure. If this is the case, the caregiver 420 will perform the required emergency measures. Subsequently, the caregiver 420 will send an alert feedback indicating the correctly identified seizure. If the patient 301 has no seizure, the caregiver 420 will send an alert feedback indicating a falsely identified seizure. In case the caregiver 420 notices a seizure, which has been undetected by the epilepsy monitoring system 100, i.e. no alert has been triggered, the caregiver uses control 404 to submit a missing alert report 326.

This description and the accompanying drawings that illustrate aspects and embodiments of the present invention should not be taken as limiting-the claims defining the protected invention. In other words, while the invention has been illustrated and described in detail in the drawings and foregoing description, such illustration and description are to be considered illustrative or exemplary and not restrictive. Various mechanical, compositional, structural, electrical, and operational changes may be made without departing from the spirit and scope of this description and the claims. In some instances, well-known circuits, structures and techniques have not been shown in detail in order not to obscure the invention. Thus, it will be understood that changes and modifications may be made by those of ordinary skill within the scope and spirit of the following claims. In particular, the present invention covers further embodiments with any combination of features from different embodiments described above and below.

The disclosure also covers all further features shown in the Figs. individually although they may not have been described in the afore or following description. Also, single alternatives of the embodiments described in the figures and the description and single alternatives of features thereof can be disclaimed from the subject matter of the invention or from disclosed subject matter. The disclosure comprises subject matter consisting of the features defined in the claims or the exemplary embodiments as well as subject matter comprising said features.

Furthermore, in the claims the word "comprising" does not exclude other elements or steps, and the indefinite article "a" or "an" does not exclude a plurality. A single unit or step may fulfil the functions of several features recited in the claims. The mere fact that certain measures are recited in mutually different dependent claims does not indicate that a combination of these measures cannot be used to advantage. The terms "essentially", "about", "approximately" and the like in connection with an attribute or a value particularly also define exactly the attribute or exactly the value, respectively. The term "about" in the context of a given numerate value or range refers to a value or range that is, e.g., within 20%, within 10%, within 5%, or within 2% of the given value or range. Components described as coupled or connected may be electrically or mechanically directly coupled, or they may be indirectly coupled via one or more intermediate components. Any reference signs in the claims should not be construed as limiting the scope.

A computer program may be stored/distributed on a suitable medium, such as an optical storage medium or a solid-state medium supplied together with or as part of other hardware, but may also be distributed in other forms, such as via the Internet or other wired or wireless telecommunication systems. In particular, e.g., a computer program can be a computer program product stored on a computer readable medium which computer program product can have computer executable program code adapted to be executed to implement a specific method such as the method according to the invention or portions thereof. Furthermore, a computer program can also be a data structure product or a signal for embodying a specific method such as the method according to the invention.

## Claims

1. Arrangement (100) for monitoring an epilepsy patient (301) during night-time, comprising
a resting location (300) equipped with a camera (302a, 302b) and a microphone (302a, 302b) for continuously gathering visual and acoustic data of the epilepsy patient (301) during night-time, wherein the resting location (300) is in a first time zone;
a supervising location equipped with a screen, a speaker and an alarm trigger (200), wherein the supervising location is in a second time zone different from the first time zone;
a caregiving location equipped with an alarm device (400), wherein the caregiving location is in the first time zone and in vicinity of the resting location; and
a central unit (101) connected to the camera (302a, 302b) and microphone (302a, 302b) of the resting location (300), to the screen and the alarm trigger of the supervising location and to the alarm of the caregiving location; wherein
the central unit (101) is arranged to route the visual and acoustic data gathered at the resting location (300) to the screen and speaker at the supervising location, and
the central unit (101) is arranged to route alarm data from the alarm trigger at the supervising location to the alarm device at the caregiving location.

2. Arrangement (100) according to claim 1, wherein the supervising location is equipped with a sensor unit (303a, 303b, 304a, 304b) for gathering sensor data, the supervising location is equipped with a sensor data presentation unit, the central unit (101) is connected to the sensor unit (303a, 303b, 304a, 304b) of the resting location (300) and to the sensor data presentation unit of the supervising location and the central unit (101) is arranged to route the sensor data gathered at the resting location (300) to the sensor data presentation unit at the supervising location.

3. Arrangement (100) according to claim 2, wherein the sensor unit (303a, 303b, 304a, 304b) comprises any of a body temperature sensor, a skin conductivity sensor, an electroencephalography sensor, an electrocardiography sensor or a combination thereof.

4. Arrangement (100) according to any one of the preceding claims, wherein the central unit (101) is arranged to monitor a status of the camera (302a, 302b), microphone (302a, 302b), screen, speaker, alarm trigger, alarm device and the connection in between, to identify a faulty status and to trigger an alarm on the alarm device at the caregiving location when the faulty status is identified.

5. Arrangement (100) according to any one of the preceding claims, further comprising a secondary supervising location equipped with a screen, a speaker and an alarm trigger, wherein the secondary supervising location is in a third time zone different from the first time zone, wherein
the central unit (101) is connected to the screen and the alarm trigger of the secondary supervising location,
the central unit (101) is arranged to route the visual and acoustic data gathered at the resting location (300) to the screen and speaker at the secondary supervising location, and
the central unit (101) is arranged to route alarm data from the alarm trigger at the secondary supervising location to the alarm device at the caregiving location.

6. Arrangement (100) according to any one of the preceding claims, wherein the central unit (101) comprises a database and is arranged to store the visual and acoustic data gathered at the resting location (300) and the alarm data routed from the alarm trigger at the supervising location to the alarm device at the caregiving location.

7. Arrangement (100) according to any one of the preceding claims comprising at least one further resting location (300) equipped with a camera (302a, 302b) and a microphone (302a, 302b) for continuously gathering visual and acoustic data of at least one further epilepsy patient (301) when sleeping, at least one further caregiving location equipped with an alarm device, wherein the at least one further caregiving location is vicinity of the at least one further resting location, and the central unit (101) is arranged to route the visual and acoustic data gathered at the at least one further resting location (300) to the screen and speaker at the supervising location and to route alarm data from the alarm trigger at the supervising location to the alarm device at the at least one further caregiving location.

8. Method of monitoring an epilepsy patient (301) during night-time by means of an arrangement (100) according to any one of the preceding claims, comprising the steps of:
the epilepsy patient (301) sleeping at the resting location;
the central unit (101) routing the visual and acoustic data of the epilepsy patient (301) gathered at the resting location (300) to the screen and speaker at the supervising location;
a medically trained person (260, 261) observing the screen and the speaker at the supervising location;
the medically trained person (260, 261) activating the alarm trigger when identifying a seizure event by interpreting the visual and acoustic data provided by the screen and speaker at the supervising location;
a caregiving person (420) perceiving an alarm provided by the alarm device at the caregiving location upon the alarm trigger activated at the supervising location; and
the caregiving person (420) transfers to the resting location (300) and attends the epilepsy patient.

9. Method according to claim 8, further comprising the step of predefining an alert criterion, wherein the medically trained person (260, 261) activates the alarm trigger when the visual and acoustic data provided by the screen and speaker at the supervising location fulfil the alert criterion.

10. Method according to claim 8 or 9, further comprising the step of the medically trained person (260, 261) activates the alarm trigger when identifying a faulty status of the arrangement.
